# EUROPEAN PATENT SPECIFICATION

(11) **EP 1 884 220 B1**
(45) Date of publication and mention of the grant of the patent: **30.03.2011**
(21) Application number: 06254011.7
(22) Date of filing: 31.07.2006
(51) Int. Cl.: A61F 2/34

(54) **Flexible acetabular cup and method of manufacture thereof**
Flexible Hüftgelenkspfanne und Verfahren zur Herstellung einer solchen
Coque acétabulaire flexible et son procédé de fabrication

(43) Date of publication of application: 06.02.2008
(73) Proprietor: BENOIST GIRARD SAS, 14201 Hérouville-Saint-Clair Cédex (FR)
(72) Inventor: Raugel, Patrick, 37530 Chargé (FR); Jones, Eric, Kildimo, County Limerick (IE)
(74) Representative: Bridge-Butler, Alan James

(56) References cited:
- EP-A1- 0 552 949
- EP-A1- 1 208 819
- EP-A2- 1 205 160
- US-A1- 2005 060 040

## Description

This invention relates to a flexible acetabular cup and a method of manufacture thereof.

Flexible acetabular cups are known, the inner bearing surfaces of which comprise a substantially part-spherical portion and two independent arms projecting therefrom and formed by a separation or opening in the rim of the cup.

Flexible acetabular cups of this type are shown in European Patent No. 0 552 949 and European Patent No. 1208 819.

Cups of this type have a polar area in a part-spherical portion which carries the main load and the part-spherical portion can be relieved over part of its surface and the arms.

With flexible cups of this type the flexibility can cause the risk of deformation at the polar contact area so that the cup deforms and creates only ring contact which is undesirable.

Prior to fitting of the cup the acetabular socket is reamed and the tolerances can leave a difference of 0 mm to 0.6 mm between the socket and the cup so it may not be a perfect fit.

It has been found that the cup can be arranged to be inserted with an interference fit of up to about 0.6 mm but above that it has been found that the interference causes too much deformation and again creates a ring contact.

The present invention is intended to provide a construction and method of making a flexible acetabular cup which overcomes the difficulties concerned.

According to the present invention a flexible acetabular cup has a substantially part-spherical portion, a pair of arms extending therefrom, an outer rim which is common to both said part-spherical portion and said pair of arms and in which said part-spherical portion extends radially outwardly from a centre point to said outer rim, and said arms are formed by a separation or opening in the outer rim characterised in that the pre-shaped distance measured from said centre point to any point on the outer rim of the arms is greater than the radius of the rim of the part-spherical portion measured from said centre point.

The separation gap or opening between the arms can be such that the width of the cup at its widest point is greater by between 0.1 mm and 1 mm than the external diameter of the part-spherical portion.

Preferably the separation gap or opening between the arms is greater by between 0.3 mm and 0.5 mm.

It has been found that the pre-stressing of the arms reduces deformation and acts to prevent the polar area of contact degenerating into ring contact.

The invention also includes a process for making the flexible acetabular cup set forth above which includes the steps of making the cup with a substantially part-spherical portion and two independent arms and then deforming it to provide the shape in which the distance from the centre point to any point on the outer rim of the arms is greater than the radius of the rim of the part-spherical portion.

The process can include the steps of:
moulding the cup from a synthetic plastics material to provide the shape with the two arms;
placing the moulded cup, whilst still warm, in a jig which has a separator, the dimensions of which are greater than the separation or opening between the arms;
applying pressure to the cup to force the separator into the separation or opening to force the arms apart;
and, allowing to cool.

Thus the separator can distort the arms by between 0.1 mm and 1 mm and preferably between 0.3 mm to 0.5 mm from the original moulded shape so that the arms pre-stress when the cup is placed in the prepared acetabulum.

A method of fitting a flexible acetabular cup into an acetabular socket is described, said method includes machining the socket to provide a part-spherical recess and then inserting a flexible cup as set forth above under pressure to cause the arms to deform towards each other so that they are pre-stressed towards each other when in place.

The invention can be performed in various ways and one embodiment will now be described by way of example and with reference to and as shown in the accompanying drawings.
Figures 1, 2 and 3 are respectively side, front and top elevations of a flexible cup of the type described in European Patent No.1208 819;
Figure 4 is a diagrammatic illustration showing how deformation of a flexible cup can cause ring contact on a ball head;
Figure 5 is a plan view of the cup shown in Figures 1, 2 and 3 showing how it can be shaped during manufacture;
Figure 6 is a diagrammatic side elevation of the cup shown in Figures 1, 2, 3 and 4 in place on a deformation jig.

The cup can be made from a single material, for example (CFR PEEK) as described in European Patent No. 1205 160, or from two or more materials as set forth in European Patent No. 1208 819.

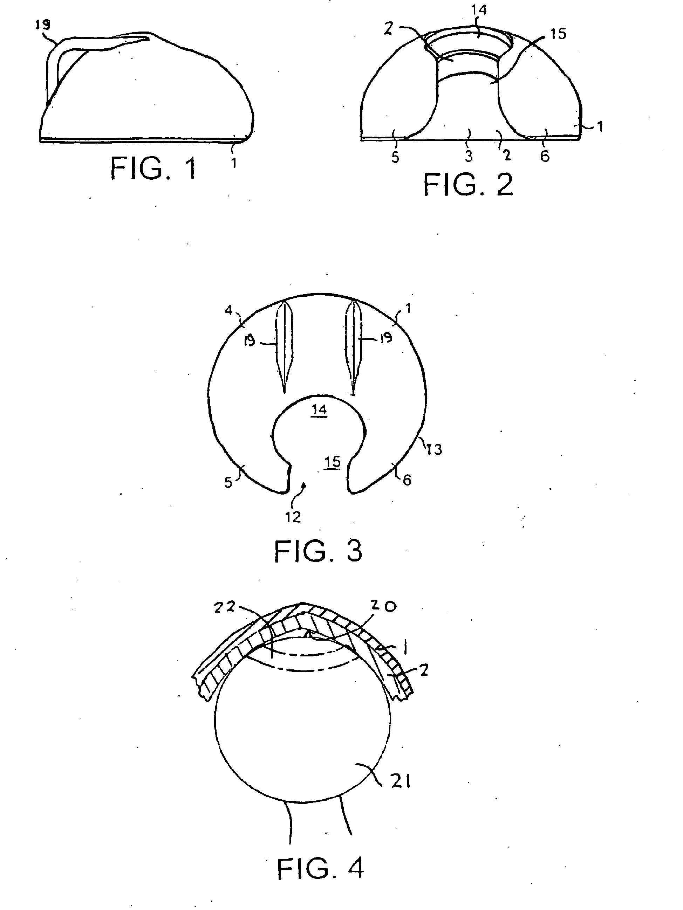

The cup shown in Figures 1, 2 and 3 is similar to that described in European Patent No. 1208 819 and comprises a backing 1 which supports a bearing liner 2. The bearing liner has a bearing surface 3 and the backing is moulded from a relatively rigid polymeric material, for example polyurethane in the form of Corothane 750D, CFR-PBT, CFR-PEEK or polymethylmethacrylate. The liner can be made from Corothane 80A and the liner and backing can be moulded together by the technique described in European Patent No.0 698 382, the liner being bonded to the backing by the moulding process.

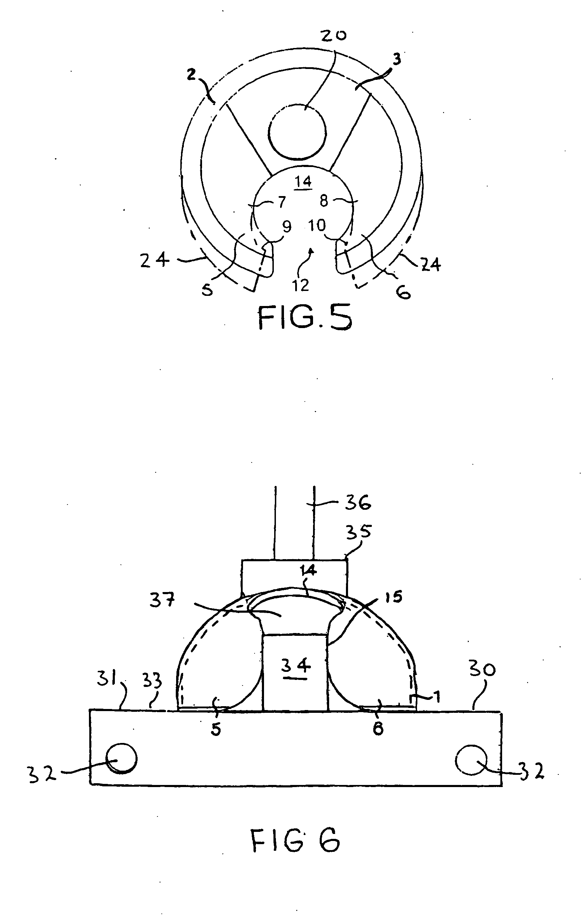

The overall shape and construction of the cup is similar to that described in European Patent No. 0 552 949, that is the main portion 4 of the backing is substantially part-spherical and there are two independent arms 5 and 6 which extend from the main part 4. The external shape of the inner bearing liner is also hemispherical and fits within the backing 1 but the inner surface of the bearing component is only hemispherical over its main portion and is relieved over the inner surfaces 7 and 8 of its arms 9 and 10, best shown in Figure 5.

If desired the inner bearing liner can be substantially hemispherical and merely carry a thin splitting line to provide the two arms but in the construction shown in the drawings the arms 5 and 6 and 9 and 10 are spaced apart to provide a gap or opening 12 between them. The arms are spaced apart about an arc on the part-spherical main portion of the backing and the liner breaking out on the rim 13 and the outer surface of the arms themselves and the main portion are together substantially part-spherical. Backing 1 thus comprises a substantially part-spherical wall having a rim 13 which is interrupted by a shaped opening to provide two spaced apart arms 5 and 6.

The main part 14 of the opening 12 is substantially semi-circular and has a mouth 15 which provides the interruption in the rim 13 and which is of smaller width than the remainder 14 of the opening. The backing 1 is therefore substantially horseshoe-shaped.

Because of its construction and the materials used the backing is sufficiently flexible to accept deformation of the acetabulum of the patient but is stiffer than the inner bearing component.

In the construction shown in the drawings the outer surface of the backing 1 does not include the projecting strakes of European Patent No. 1208 819 and the surface is substantially part-spherical apart from a pair of projecting fins 19 which extend in spaced apart parallel chordal directions.

The outer surface of the backing is plasma sprayed with hydroxyapatite (HA) coating which is osteo-conductive and stimulates bone growth. The cup can also be made as set forth in European Patent No. 1647 242 and have a metal backing layer, for example titanium, tantalum or niobium or even pure PEEK, to produce a barrier between composite material and the bone cells.

In order to insert the acetabular cup into an acetabulum, the bone is first prepared to the appropriate shape and a series of triangular grooves are carved into it by the surgeon which will line up with and accept fins 19.

The cup is now placed in position and tapped, the fins 19 holding it accurately as required. It has been found that the pressure of the ball of the femoral prosthesis or the natural ball shape of the bone acts to pressure the cup to hold it in position whilst bone growth takes place.

With this arrangement it is desirable to provide certain tolerances but it has been found that there is a risk of deformation at the polar contact area (indicated by reference numeral 20 in Figure 5) so that the cup deforms and creates only ring contact which is undesirable and provides excessive wear and high frictional torque. This can take place with cups as described above or cups having plain outer surfaces or made from a single material. In figure 4 the deformation is exaggerated to explain the problem.

The effect of the deformation is shown diagrammatically in Figure 4. The polar area 20 of the cup deforms upwardly so that contact with the ball, indicated by reference numeral 21, is reduced to a ring or line contact, indicated by chain lines 22. Such ring contact is undesirable and provokes wear on the surface as referred to above.

Figure 5 is a plan view of the construction shown in Figures 1, 2 and 3 and shows how the arms 5 and 6 can be pre-shaped which causes them to be pre-stressed inwardly towards opening 12 when fitted into a prepared part-spherical acetabular socket.

The cup is moulded initially to the shape shown in full lines in Figures 1, 2, 3 and 5. It is then deformed to the shape indicated by chain lines 24 in Figure 5. In order to achieve the deformation a jig 30 is employed and which comprises a base 31 provided with cooling water channels 32. The upper surface 33 of the base is substantially flat or shaped to accept the outer periphery of the rim of the cup with which it is to be used. A separator block 34 is provided on the base 30 and above it is located a pressure pad 35 which can be pressed downwardly by a pressure arm 36. The cup is located on a domed boss 37 also provided on the base 30.

After the cup has been initially moulded, but whilst it is still warm, it is placed in the jig 30. The width of the block 34 is greater than the width of the opening 12 of the moulded cup by between 0.1 mm and 1 mm and preferably between 0.3 mm and 0.5 mm. With the cup placed on the block 34 pressure is applied to the pressure pad 35 which forces the cup over the block thus distorting the arms 5 and 6 so that they are pressured outwardly to the position shown by broken lines 24 in Figure 5. It will be appreciated that the distances shown in Figure 5 are exaggerated to explain the process.

With the cup held in this position it is then allowed to cool assisted by the water cooling in the channels 32.

The removed cup is of the shape shown in chain lines in Figure 5.

In order to install the cup in a prosthetic socket the socket is machined to a hemispherical shape. It will be appreciated that the distortion has caused the arms to expand slightly in relation to the remaining portion of the cup so that it is no longer part-spherical but of a slightly deformed shape. When the cup is now placed in the part-spherical machined socket the arms are pressed towards each other so that the cup is pre-pressurised.

It has been found that with this pre-pressurisation and with an interference fit effect of up to 0.6 mm deformation of the cup in the polar area is prevented as is the creation of ring contact.

It will be appreciated that in the description of the device and apparatus shown above a particular embodiment of cup is referred to but the invention can be applied to any cup of the type which has the two separated arms and which can have a variety of types of construction or shape.

## Claims

1. A flexible acetabular cup having a part-spherical portion (4), a pair of arms (5), (6) extending therefrom, an outer rim (13) which is common to both said part-spherical portion (4) and said pair of arms (5), (6) and in which said part-spherical portion (4) extends radially outwardly from a centre point to said outer rim (13), and said arms (5), (6) are formed by a separation or opening in the outer rim (13) **characterised in that** the pre-shaped distance measured from said centre point to any point on the outer rim (13) of the arms (5), (6) is greater than the radius of the rim (13) of the part-spherical portion measured from said centre point.

2. A flexible acetabular cup as claimed in claim 1 in which the separation or opening (12) between the arms (5), (6) is such that an external diameter of the cup at its widest point is greater by between 0.1 mm and 1 mm than the external diameter of the part-spherical portion (4)

3. A flexible acetabular cup as claimed in claim 2 in which the separation or opening (12) between the arms (5), (6) is such that an external diameter of the cup at its widest point is greater by between 0.3 mm and 0.5 mm than the external diameter of the part-spherical portion (4).

4. A flexible acetabular cup as claimed in any one of the preceding claims in which the cup is made from a single material or from two or more materials.

5. A flexible acetabular cup as claimed in claim 4 in which the cup is made from (CFR PEEK).

6. A flexible acetabular cup as claimed in claim 4 in which the cup is made from two materials and has a bearing liner (2) and a backing (1) moulded from a rigid polymeric material, for example polyurethane in the form of Corothane 80A.

7. A flexible acetabular cup as claimed in claim 6 in which the backing material is Corothane 750D, CFR-PBT, CFR-PEEK or polymethylmethacrylate.

8. A flexible acetabular cup as claimed in claim 4 in which the cup is made from two materials and has a metal or synthetic plastics material backing layer (1).

9. A flexible acetabular cup as claimed in claim 8 in which the backing layer (1) is titanium, tantalum or niobium.

10. A flexible acetabular cup as claimed in claim 8 in which the synthetic plastics material backing layer (1) is made from pure PEEK.

11. A flexible acetabular cup as claimed in any one of claims 6 to 10 in which the bearing liner(2) is made from a composite material.

12. A flexible acetabular cup as claimed in any one of claims 6 to 10 which has an outer surface which has been plasma sprayed with a hydroxyapatite (HA) coating

13. A process for making the flexible acetabular cup as claimed in claims 1 to 12 **characterised by** including the steps of :
making the cup with a substantially part-spherical portion (4) and two independent arms (5), (6) and then deforming it to provide the shape in which the distance from the centre point to any point on the outer rim (13) of the arms (5), (6) is greater than the radius of the rim (13) of the part spherical portion (4).

14. A process for making the flexible acetabular cup as set forth in claim 13 **characterised by** including the steps of:
moulding the cup from a synthetic plastics material to provide the shape with the two arms (5, 6);
placing the moulded cup, whilst still warm, in a jig (30) which has a separator (34), the dimensions of which are greater than the separation or opening (12) between the arms (5, 6);
applying pressure to the cup to force the separator (34) into the separation or opening (12) to force the arms (5, 6) apart;
and, allowing to cool.

15. A process as claimed in claim 14 in which the separator (34) is used to force the arms (5, 6) apart between 0.1 mm and 1 mm from the original moulded shape.

16. A process as claimed in claim 15 in which the arms (5, 6) are forced apart between 0.3 mm and 0.5 mm from the original moulded shape.

## Patentansprüche

1. Flexible Hüftgelenkspfanne mit einem teilsphärischen Teil (4), einem Paar Arme (5),(6), die sich daraus erstrecken, einem äußeren Rand (13), der sowohl dem teilsphärischen Teil (4) und dem Paar Arme (5),(6) gemein ist, und wobei der teilsphärische Teil (4) sich von einem Mittelpunkt aus radial auswärts zu dem äußeren Rand (13) erstreckt, und wobei die Arme (5), (6) von einer Unterbrechung oder Öffnung im äußeren Rand (13) gebildet werden, **dadurch gekennzeichnet, dass** der vorgeformte Abstand, gemessen von dem Mittelpunkt aus bis zu einem beliebigen Punkt auf dem äußeren Rand (13) der Arme (5),(6), größer ist als der Radius des Randes (13) des teilsphärischen Teils, gemessen von dem Mittelpunkt aus.

2. Flexible Hüftgelenkspfanne nach Anspruch 1, wobei die Unterbrechung oder Öffnung (12) zwischen den Armen (5), (6) derart ist, dass ein Außendurchmesser der Pfanne arm ihrer weitesten Steile um zwischen 0,1 mm und 1 mm größer ist als der Außendurchmesser des teilsphärischen Teils (4).

3. Flexible Hüftgelenkspfanne nach Aspruch 2, wobei die Unterbrechung oder Öffnung (12) zwischen den Armen (5), (6) derart ist, dass ein Außendurchmesser der Pfanne an ihrer weitesten Stelle um zwischen 0,3 mm und 0,5 mm größer ist als der Außendurchmesser des teilsphärischen Teils (4).

4. Flexible Hüftgelenkspfanne nach einem der vorangehenden Ansprüche, wobei die Pfanne aus einem einzigen Material oder aus zwei oder mehr Materialien besteht.

5. Flexible Hüftgelenkspfanne nach Anspruch 4, wobei die Pfanne aus (CFR PEEK) besteht.

6. Flexible Hüftgelenkspfanne nach Aspruch 4, wobei die Pfanne aus zwei Materialien besteht und eine Lagerauskleidung (2),sowie einen Träger (1) aufweist, der aus einem starren Polymermaterial geformt ist, zum Beispiel Polyurethan in Form von Corothane 80A.

7. Flexible Hüftgelenkspfanne nach Anspruch 6, wobei das Trägermaterial Corethane 750D, CFR-PBT, CFR-PEEK oder Polymethylmethacrylat ist.

8. Flexible Hüftgelenkspfanne nach Anspruch 4, wobei die Pfanne aus zwei Materialien besteht und eine Metall- oder Kunststoffmaterial-Trägerschicht (1) aufweist.

9. Flexible Hüftgelenkspfanne nach Anspruch 8, wobei die Trägerschicht (1) Titan, Tantal oder Niob ist.

10. Flexible Hüftgelenkspfanne nach Anspruch 8, wobei die Kunststoffmaterial-Trägerschicht (1) aus reinem PEEK besteht.

11. Flexible Hüftgelenkspfanne nach einem der Anspruche 6 bis 10, wobei die Lagerauskleidung (2) aus einem Verbundmaterial besteht.

12. Flexible Hüftgelenkspfanne nach einem der Ansprüche 6 bis 10, die eine äußere Oberfläche aufweist, die mit einer Hydroxylapatit (HA)-Beschichtung plasmagesprüht worden ist.

13. Verfahren zur Herstellung der flexiblen Hüftgelenkspfanne nach den Ansprüchen 1 bis 12, **dadurch gekennzeichnet, dass** es die Schritte einschließt:
Herstellen der Pfanne mit einem im Wesentlichen teilsphärischen Teil (4) und zwei unabhängigen Armen (5), (6) und dann Verformen derselben, um die Gestalt bereitzustellen, bei welcher der Abstand vom Mittelpunkt bis zu einem beliebigen Punkt auf dem äußeren Rand (13) der Arme (5), (6) größer ist als der Radius des Randes (13) des teilsphärischen Teils (4).

14. Verfahren zur Herstellung der flexible Hüftgelenkspfanne nach Anspruch 13, **dadurch gekennzeichnet, dass** es die Schritte einschließt:
Formen der Pfanne aus einem Kunststoffmaterial, um die Gestalt mit den zwei Armen (5), (6) bereitzustellen;
Platzierten der geformten Pfanne, wähnend sie noch warm ist, in einer Einspannvorrichtung (30), die einen Separator (34) aufweist, dessen Abmessungen größer sind als die Unterbrechung oder Öffnung (12) zwischen den Armen (5), (6);
Aufbringen eines Drucks auf die Pfanne, zum den Separator (34) in die Unterbrechung oder Öffnung (12) zu drücken, um die Arme (5), (6) auseinander zu drucken;
und Abkühlen lassen.

15. Verfahren nach Anspruch 14, wobei der Separator (34) benutzt wird, um die Arme zwischen 0,1 mm und 1 mm aus der ursprünglichen geformten Gestalt auseinander zu drücken.

16. Verfahren nach Anspruch 15, wobei die Arme (5), (6) zwischen 0,3 mm und 0,5 mm aus der ursprünglichen geformten Gestalt auseinander gedrückt werden.

## Revendications

1. Cupule cotyloïdienne flexible ayant une partie partiellement sphérique (4), une paire de bras (5) (6) s'étendant depuis celle-ci, un bord extérieur (13) commun avec ladite partie partiellement sphérique (4) ainsi qu'avec ladite paire de bras (5), (6) et dans laquelle ladite partie partiellement sphérique s'étend radialement vers l'extérieur depuis un point central jusqu'audit bord extérieur (3), et lesdits bras (5), (6) sont formés par une séparation ou une ouverture dans le bord extérieur (13), **caractérisée en ce que** la distance préformée mesurée dudit point central à n'importe quel point sur le bord extérieur (13) des bras (5, 6) est plus grande que le rayon du bord (13) de la partie partiellement sphérique mesuré depuis ledit point central.

2. Cupule cotyloïdienne flexible selon la revendication 1, dans laquelle la séparation ou ouverture (12) entre les bras (5), (6) est telle qu'un diamètre extérieur de la cupule dans sa plus grande largeur est plus grand de 0,1 mm à 1 mm que le diamètre extérieur de la partie partiellement sphérique (4).

3. Cupule cotyloïdienne flexible selon la revendication 2, dans laquelle la séparation ou ouverture (12) entre les bras (5), (6) est telle qu'un diamètre extérieur de la cupule dans sa plus grande largeur est plus grand de 0,3 mm à 0,5 mm que le diamètre extérieur de la partie partiellement sphérique (4).

4. Cupule cotyloïdienne flexible selon l'une quelconque des revendications précédentes, dans laquelle la cupule est faite d'une seule matière ou de deux ou plus de deux matières.

5. Cupule cotyloïdienne flexible selon la revendication 4, dans laquelle la cupule est en polyétheréthercétone renforcé de fibres de carbone.

6. Cupule cotyloïdienne flexible selon la revendication 4, dans laquelle la cupule est faite de deux matières et possède un revêtement d'appui (2) et un renforcement (1) en polymère rigide, par exemple en polyuréthane sous la forme de Corethane 80A.

7. Cupule cotyloïdienne flexible selon la revendication 6, dans laquelle la matière de renforcement est du Corethane 80A, du polybutylène téréphtalate renforcé de fibres de carbone, du polyétheréthercétone renforcé de fibres de carbone ou du polymétacrylate de méthyle.

8. Cupule cotyloïdienne flexible selon la revendication 4, dans laquelle la cupule est faite de deux matières et comporte une couche de renforcement (1) en métal ou en matière plastique synthétique.

9. Cupule cotyloïdienne flexible selon la revendication 8, dans laquelle la couche de renforcement (1) est en titane, en tantale ou en niobium.

10. Cupule cotyloïdienne flexible selon la revendication 8, dans laquelle la couche de renforcement (1) en matière plastique synthétique est en polyétheréthercétone pur.

11. Cupule cotyloïdienne flexible selon l'une quelconque des revendications 6 à 10, dans laquelle le revêtement d'appui (2) est en matériau composite.

12. Cupule cotyloïdienne flexible selon l'une quelconque des revendications 6 à 10, ayant une surface extérieure ayant reçu un revêtement d'hydroxyapatite (HA) projeté par plasma.

13. Procédé pour fabriquer la cupule cotyloïdienne selon les revendications 1 à 12, **caractérisé en ce qu'**il comprend les étapes consistant à :
doter la cupule d'une partie sensiblement partiellement sphérique (4) et de deux bras indépendants (5), (6), puis déformer celle-ci pour créer la forme dans laquelle la distance du point central à n'importe quel point sur le bord extérieur (13) des bras (5, 6) est plus grande que le rayon du bord (13) de la partie partiellement sphérique (4).

14. Procédé pour fabriquer la cupule cotyloïdienne selon la revendication 13, **caractérisé en ce qu'**il comprend les étapes consistant à :
mouler la cupule à l'aide d'une matière plastique synthétique pour créer la forme avec les deux bras ;
placer la cupule moulée, tandis qu'elle est encore chaude, dans un gabarit (30) pourvu d'un séparateur (34) dont les dimensions sont plus grandes que la séparation ou l'ouverture (12) entre les bras (5, 6) ;
exercer une pression sur la cupule pour contraindre le séparateur (34) à venir dans la séparation ou l'ouverture (12) afin d'amener les bras (5, 6) à s'écarter l'un de l'autre ;
et laisser refroidir.

15. Procédé selon la revendication 14, dans lequel le séparateur (34) sert à amener les bras (5, 6) à s'écarter l'un de l'autre de 0,1 mm à 1 mm par rapport à la forme d'origine obtenue par moulage.

16. Procédé selon la revendication 15, dans lequel les bras (5, 6) sont amenés à s'écarter l'un de l'autre de 0,3 mm à 0,5 mm par rapport à la forme d'origine obtenue par moulage.
